Europäisches Patentamt

European Patent Office

Office européen des brevets

(11) Veröffentlichungsnummer: **0 153 570**
**A1**

# EUROPÄISCHE PATENTANMELDUNG

(21) Anmeldenummer: **85100386.3**

(22) Anmeldetag: **16.01.85**

(51) Int. Cl.⁴: **A 61 B 3/14**
**G 03 B 17/48**

(30) Priorität: **30.01.84 DE 8402582 U**

(43) Veröffentlichungstag der Anmeldung:
**04.09.85 Patentblatt 85/36**

(84) Benannte Vertragsstaaten:
**BE DE FR GB IT LU NL**

(71) Anmelder: **Futronic GmbH**
**Waldesch 29**
**D-7992 Tettnang 1(DE)**

(72) Erfinder: **Richard, Gisbert. Dr.med.**
**Herrenberger Strasse 36**
**D-7400 Tübingen(DE)**

(72) Erfinder: **Funk, Gerhard**
**Waldesch 29**
**D-7992 Tettnang 1(DE)**

(74) Vertreter: **Kador . Klunker . Schmitt-Nilson . Hirsch**
**Corneliusstrasse 15**
**D-8000 München 5(DE)**

(54) Videoangiographie-Kameraanordnung.

(57) Videoangiographie-Kameraanordnung zur angiographischen Aufzeichnung mittels Videorecorder, mit einer Funduskamera (11) zum Beleuchten der Netzhaut eines zu untersuchenden Auges (39) und zum Abbilden der beleuchteten Netzhaut, wobei an dem Gehäuse der Funduskamera (11) das Gehäuse einer restlichtempfindlichen Videokamera (13) starr befestigt ist und zwischen die Ausgangsseite des Aufnahmeobjektivs (41) der Funduskamera (11) und die Eingangsseite des Aufnahmeobjektivs (19) der Videokamera (13) ein diese beiden Objektive optisch verbindender Strahlengangkoppler (21) eingefügt ist.

FIG. 2

futronic GmbH
Waldesch 29
7992 Tettnang 1

— 1 —

(u.Z.K22 2745/6)

## Videoangiographie-Kameraanordnung

Bei der Angiographie handelt es sich um ein Diagnoseverfahren, mit dem der Zustand und das Verhalten von Gefäßen im Bereich der Augennetzhaut beobachtet werden können, um beispielsweise Gefäßerkrankungen im Netzhautbereich oder Erkrankungen wie Bluthochdruck anhand ihrer Auswirkungen auf diese Gefäße diagnostizieren zu können. Die Angiographie ist außerdem bei wissenschaftlichen Untersuchungen einsetzbar, die dem Verhalten von Blutgefäßen dienen, da das Auge das einzige Fenster des Menschen zur Beobachtung von Blutgefäßen darstellt.

Der Nachweis von Veränderungen der Gefäße sowie benachbarter Gewebestrukturen wird erleichtert, wenn man nach intravenöser Eingabe von Natriumfluorescein nach Einwirken von kurzwelligem Licht das in einem charakteristischen Spektralbereich emittierte langwellige Licht beobachtet (Fluoreszenzangiographie).

Bei der herkömmlichen Sequenzangiographie werden mittels einer Funduskamera photographisch Einzelbilder hergestellt. Da die Reaktion der Netzhautgefäße auf das injizierte Kontrastmittel nur über eine relativ kurze Zeit andauert, hat man die Bildfrequenz durch Einsatz von Schnellblitzgeneratoren zu erhöhen versucht. Das Blitzen mit relativ hoher Lichtenergie wird von den Patienten als außerordentlich unangenehm empfunden und löst auch häufiges Blinzeln aus.

Eine Fluoreszenzangiographie mit fortlaufender Aufzeichnung hat man in Form der Fluoreszenzkinematographie versucht. Die dafür erforderlichen sehr hohen Lichtintensitäten werden von den Patienten nicht ertragen und sind daher in ihrer Anwendung praktisch auf Tierversuche beschränkt.

Erste Versuche einer Videoangiographie haben bisher nicht zu zufriedenstellenden Ergebnissen geführt. Auch hierbei benötigte man bisher zu hohe Lichtintensitäten, um von der Netzhaut reflektiertes oder emittiertes Licht in ausreichender Menge auf das Target einer Videokamera zu bekommen.

Die Probleme der herkömmlichen Angiographiemethoden werden überwunden durch Einsatz einer Videoangiographie-Kameraanordnung, die eine Funduskamera zum Beleuchten der Netzhaut eines zu untersuchenden Auges und zum Abbilden der beleuchteten Netzhaut aufweist und die dadurch gekennzeichnet ist, daß an dem Gehäuse der Funduskamera das Gehäuse einer restlichtempfindlichen Videokamera starr befestigt ist und daß zwischen die Ausgangsseite des Aufnahmeobjektivs der Funduskamera und die Eingangsseite des Aufnahmeobjektivs der Videokamera ein diese beiden Objektive optisch verbindender Strahlengangkoppler eingefügt ist.

Bei einer bevorzugten Weiterbildung dieser Anordnung weist der Strahlengangkoppler ein Rohr auf, an dessen beiden Enden je ein anderen Endes offenes Winkelendstück und je ein Umlenkspiegel anordnet sind, wobei ein Winkelendstück an der Ausgangsseite des Aufnahmeobjektivs der Funduskamera und das andere Winkelendstück an der Eingangsseite des Aufnahmeobjektivs der Videokamera festgelegt ist. Die Öffnung des an der Fundus-

kamera festgelegten Winkelendstücks kann in der bei herkömmlicher Verwendung der Funduskamera die Filmebene bildenden Ebene oder an der Ausgangsseite eines Okulars der Funduskamera festgelegt sein.

Bei einer besonders bevorzugten Ausführungsform weist das an der Funduskamera angeordnete Winkelstück hinter dem Umlenkspiegel eine weitere Öffnung auf, an der sich das Objektiv einer photographischen Kamera befindet. Es können dann neben den video- graphischen Aufzeichnungen auch photographische Auf- zeichnungen durchgeführt werden. Zu diesem Zweck kann der Umlenkspiegel in diesem Winkelendstück als halbdurchlässiger Spiegel ausgebildet sein, der die vom Aufnahmeobjektiv der Funduskamera kommenden Lichtstrahlen sowohl zum anderen Winkelendstück als auch zur photographischen Kamera gelangen läßt, oder dieser Umlenkspiegel kann verschwenkbar ausgebildet sein, so daß er aus dem Strahlengang zwischen dem Aufnahmeobjektiv der Funduskamera und dem Aufnahmeobjektiv der photographischen Kamera herausschwenkbar ist. Die Herausschwenkbarkeit dieses Umlenkspiegels ist erforderlich, wenn er lichtundurchlässig ist, kann aber auch bei Verwendung eines halbdurchlässigen Umlenkspiegels von Vorteil sein, wenn man an der photographischen Kamera eine größere Lichtmenge verfügbar haben möchte als sie vom halbdurchlässigen Spiegel durchgelassen wird.

Die Erfindung wird nun anhand einer Ausführungsform näher erläutert. In der Zeichnung zeigen:

Fig. 1     eine schematisierte Perspektivdarstellung einer
           Ausführungsform der neuen Videoangiographie-Kameraanordnung;

Fig. 2     eine schematisierte Schnittdarstellung mit den
           wichtigsten Teilen der neuen Videoangiographie-
           Kameraanordnung.

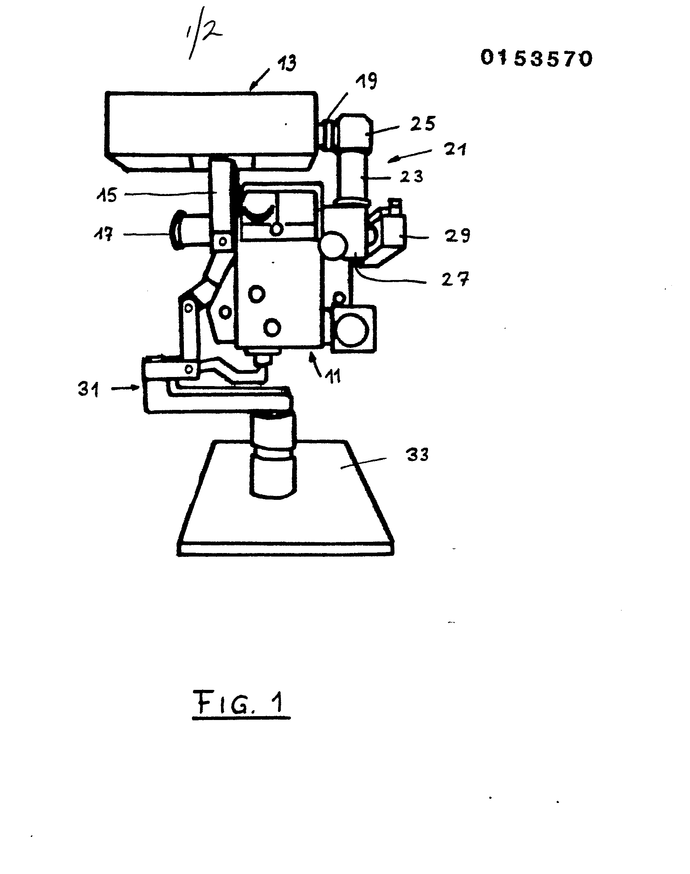

Fig. 1 zeigt eine bevorzugte Ausführungsform der neuen Videoangiographie-Kameraanordnung (im folgenden kurz Kameraanordnung genannt). An einer Funduskamera 11 ist eine Videokamera
13 mittels eines Tragfußes 15 starr verbunden. Dieser ist mit
derjenigen Vorderseite der Funduskamera 11 verbunden, aus
welcher ein zum Auge des Untersuchten gerichteter Tubus 17
hervorragt. Die Videokamera 13 ist derart auf der Funduskamera 11 befestigt, daß das Aufnahmeobjektiv 19 der Videokamera 13 in etwa über der Rückseite der Funduskamera 11 liegt.

Zwischen die Rückseite der Funduskamera 11 und das Aufnahmeobjektiv 19 der Videokamera 13 ist ein Strahlengangkoppler 21
gefügt, mit dem die Lichtstrahlen, die nach ihrem Eintritt
in den Tubus 17 durch das Aufnahmeobjektiv der Funduskamera hindurchgelangt sind, zum Aufnahmeobjektiv 19 der
Videokamera 13 gelenkt werden. Der Strahlengangkoppler 21
umfaßt ein vertikal verlaufendes Rohrstück 23, an dessen
oberem bzw. unteren Ende je ein als Winkelendstück dienendes
oberes Umlenkspiegelgehäuse 25 bzw. unteres Umlenkspiegelgehäuse 27 angeordnet sind. Das obere Umlenkspiegelgehäuse
25 ist einerseits zum Rohrstück 23 hin und andererseits
zum Aufnahmeobjektiv 19 hin geöffnet. Das untere Umlenkspiegelgehäuse 27 ist einerseits zur Rückseite der Funduskamera 11 und andererseits zum unteren Ende des Rohrstücks
37 hin geöffnet. Das untere Umlenkspiegelgehäuse 27 weist außerdem eine der Rückseite der Funduskamera 11 entgegengesetzte dritte Öffnung auf, in der das Aufnahmeobjektiv einer photographischen Sequenzkamera 29 angeordnet ist.

An der Vorder- und an der Unterseite der Funduskamera 11 sowie am Tragfuß 15 ist ein Stativ 31 befestigt, mit dessen Hilfe die Kameraanordnung auf einer Tragplatte 33 verdrehbar und verschwenkbar montiert ist.

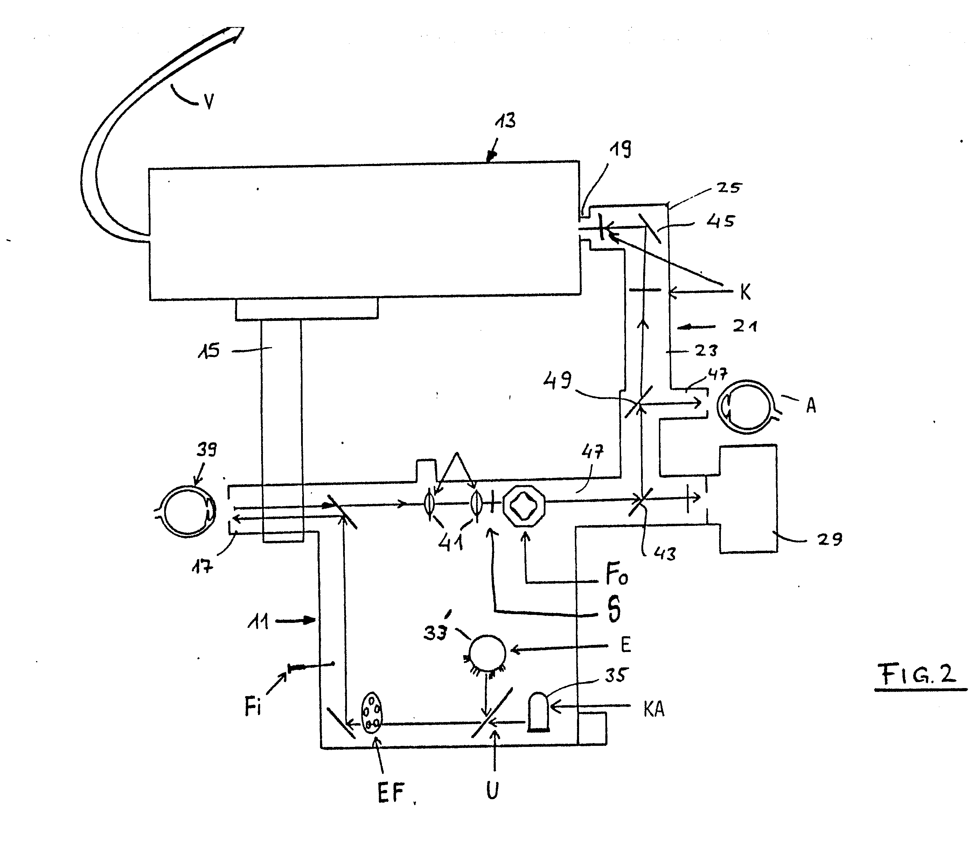

Anhand von Fig. 2 wird nun eine nähere Erläuterung von Funktionsteilen der Kameraanordnung vorgenommen.

In der Funduskamera 11 erzeugtes Erregerlicht von einer Dauerlichtquelle 33' oder einer Blitzlichtquelle 35 wird über Umlenkspiegel und nach dem Durchlaufen eines Erregerfilters 37 auf das vor der Außenöffnung des Tubus 17 befindliche zu untersuchende Auge gerichtet. Von der Netzhaut des Auges 39 kommendes Licht gelangt durch den Tubus 17 auf ein Funduskamera-Aufnahmeobjektiv 41. Hinter dessen Ausgang befindet sich eine Funduskamera-Ausgangsöffnung 47, durch welche das Licht in das untere Umlenkspiegelgehäuse 27 des Strahlengangkopplers 21 gelangt. Über einen unteren Umlenkspiegel 43 im unteren Umlenkspiegelgehäuse27 und einen oberen Umlenkspiegel 45 im oberen Umlenkspiegelgehäuse 25 gelangt das Licht dann auf das Aufnahmeobjektiv 19 der restlichtempfindlichen Videokamera 13.

Die Sequenzkamera 29 befindet sich an der weiteren Öffnung des unteren Umlenkspiegelgehäuses 27 in direkter Strahlengangverlängerung des von dem Funduskamera-Aufnahmeobjektiv 41 kommenden Lichtes.

Das Rohrstück 23 des Strahlengangkopplers 21 weist zwischen

unterem und oberen Umlenkspiegelgehäuse 27 bzw. 25 einen Okulartubus  47 auf, mit dessen Hilfe das Bild der Netzhaut des zu untersuchenden Auges 39 betrachtet werden kann. Auf der Höhe der Öffnung dieses Okulartubus 47 befindet sich im Rohrstück 23 ein mittlerer Umlenkspiegel 49, mit dem das vom unteren Umlenkspiegel 43 kommende Licht in den Okulartubus 47 gelenkt wird.

Der untere Umlenkspiegel 43 und der mittlere Umlenkspiegel 49 können entweder als halbdurchlässige Spiegel oder als aus dem Strahlengang herausschwenkbare Spiegel ausgebildet sein. Letzteres empfiehlt sich besonders, wenn man mit besonders wenig Erregerlicht auskommen möchte und durch die halbdurchlässigen Spiegel verursachte Verluste an auf das Target der Videokamera gelangendem Licht ausschalten möchte.

Auch bei Ausbildung von unterem und mittleren Umlenkspiegel 43 bzw. 49 als halbdurchlässiger Spiegel kann man diese beiden Spiegel als herausschwenkbare Spiegel ausbilden, um bei besonderem Bedarf Lichtverluste infolge der halbdurchlässigen Spiegel zu vermeiden, die in anderen Fällen tragbar sind.

Die Dauerlichtquelle 33' dient der Beleuchtung  bei videoangiographischer Benutzung der Kameraanordnung. Die Blitzlichtquelle 35 dagegen dient zur Beleuchtung  bei photographischer Benutzung der Kameraanordnung.

Man kann den Okulartubus 47 mit dem dafür erforderlichen mittleren Umlenkspiegel 49 weglassen und stattdessen den Sucher der Sequenzkamera 29 als "Okular" der Kameraanordnung verwenden. Dies verringert nicht nur den technischen Gesamtaufwand sondern verringert auch bei der videoangiographischen Benutzung der Kameraanordnung die Lichtverluste auf dem Weg zum Aufnahmeobjektiv 19 der Videokamera
13.

*8*

BEZUGSZEICHENLISTE

A:      Auge des Untersuchers

E:      Erregerlicht

EF:     Erregerfilter

Fi :     Fixierpunkt

Fo :     Fokussierung

K :      Korrekturlinsen

KA:     Kamerablitz

S:      Sperrfilter

U:      Umlenkspiegel

V:      Videosignal

futronic GmbH

Waldesch 29

7992 Tettnang 1


u.Z.: K 22 2745/6sw                16.Januar 1985


Videoangiographie-Kameraanordnung
_____


Ansprüche


1.  Videoangiographie-Kameraanordnung zur angiographischen
Aufzeichnung mittels Videorecorder, mit einer Funduskamera
zum Beleuchten der Netzhaut eines zu untersuchenden Auges
und zum Abbilden der beleuchteten Netzhaut,
dadurch g e k e n n z e i c h n e t,
daß an dem Gehäuse der Funduskamera (11)  das Gehäuse
einer restlichtempfindlichen Videokamera (13) starr
befestigt ist
und daß zwischen die Ausgangsseite des Aufnahmeobjektivs (41)
der Funduskamera (11) und die Eingangsseite des Aufnahmeobjektivs (19) der Videokamera (13) ein diese beiden Objektive
optisch verbindender Strahlengangkoppler (21) eingefügt ist.


2.  Videoangiographie-Kameraanordnung nach Anspruch 1,
dadurch gekennzeichnet,
daß der Strahlengangkoppler (21) ein Rohr  (23) aufweist,
an dessen beiden Enden je ein anderen Endes offenes
Winkelendstück (25,27) und je ein Umlenkspiegel (43,45)
angeordnet sind
und daß ein Winkelendstück (27) an der Ausgangsseite des
Aufnahmeobjektivs (41) der Funduskamera (11) und das

andere Winkelstück (25) an der Eingangsseite des
Aufnahmeobjektivs (19) der Videokamera (13) festgelegt ist.

3.    Videoangiographie-Kameraanordnung nach Anspruch 2,
dadurch gekennzeichnet,
daß die Öffnung      des an der Funduskamera (11)
festgelegten Winkelendstücks (27) in der Filmebene
der Funduskamera (11) angeordnet ist.

4.    Videoangiographie-Kameraanordnung nach Anspruch 2,
dadurch gekennzeichnet,
daß die Öffnung      des an der Funduskamera (11)
festgelegten Winkelendstücks (27)  an der Ausgangsseite
eines Okulars      der Funduskamera (11) festgelegt ist.

5.    Videoangiographie-Kameraanordnung nach einem
der Ansprüche 2 bis 4,
dadurch gekennzeichnet,
daß der zur Ausgangsseite des Aufnahmeobjektivs (41)
der Funduskamera (11) weisende Umlenkspiegel (43)
als halbdurchlässiger Spiegel ausgebildet ist,
daß das den halbdurchlässigen Spiegel (43) aufnehmende
Winkelendstück (27) auf der vom Aufnahmeobjektiv (41)
der Funduskamera abgewandten Seite des halbdurchlässigen
Spiegels (43) eine weitere Öffnung      aufweist
und daß an diesem Winkelendstück (27)  eine photographische
Kamera (29) festgelegt ist, deren Aufnahmeobjektiv
an der weiteren Öffnung      liegt.

6.    Videoangiographie-Kameraanordnung nach Anspruch 5,
dadurch gekennzeichnet,
daß der zur Ausgangsseite des Aufnahmeobjektivs (41)
der Funduskamera ( 11) weisende Umlenkspiegel (43) aus dem Strahlengang zwischen dem Aufnahmeobjektiv (41)
der Funduskamera (11) und dem Aufnahmeobjektiv
der photographischen Kamera (29) herausschwenkbar
ist.

7.    Videoangiographie-Kameraanordnung nach Anspruch 6,
dadurch gekennzeichnet,
daß der verschwenkbare Umlenkspiegel (47) undurchlässig
ist.

1/2

0153570

FIG. 1

FIG.2

0153570

**Europäisches Patentamt**

**EUROPÄISCHER RECHERCHENBERICHT**

0153570
Nummer der Anmeldung

EP 85100386.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | US - A - 3 915 564 (J.C. URBAN) | 1 | A 61 B 3/14 |
| A | * Gesamt * | 2,5,6 | G 03 B 17/48 |
| | -- | | |
| A | US - A - 4 146 310 (Y. KOHAYAKAWA et al.) | 1,2,4-7 | |
| | * Gesamt; insbesonders Fig. 1; Spalte 3, Zeilen 13-58 * | | |
| | -- | | |
| A | US - A - 4 253 743 (I. MATSUMURA) | 1,2,5-7 | |
| | * Gesamt; insbesonders Fig. 1; Spalte 3, Zeilen 4-45 * | | |
| | ---- | | |

| RECHERCHIERTE SACHGEBIETE (Int Cl 4) |
|---|
| A 61 B 3/00 |
| G 02 B 21/00 |
| G 03 B 17/00 |

Der vorliegende Recherchenbericht wurde für alle Patentansprüche erstellt.

| Recherchenort | Abschlußdatum der Recherche | Prüfer |
|---|---|---|
| WIEN | 10-05-1985 | LUDWIG |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82